# EUROPEAN PATENT APPLICATION

(11) **EP 4 846 934 A1**
(43) Date of publication of application: **02.12.2026**
(21) Application number: 25178932.7
(22) Date of filing: 27.05.2025
(51) Int. Cl.: A61B 5/055, A61B 5/00

(54) **PATIENT ENGAGEMENT TOOL**

(71) Applicant: Koninklijke Philips N.V., 5656 AE Eindhoven (NL)
(72) Inventor: HELLE, Michael Günter, Eindhoven (NL); NAUTS, Sanne, Eindhoven (NL); FRERKING, Lena Christina, 5656AG Eindhoven (NL); WIEMKER, Rafael, Eindhoven (NL); VAN EE, Raymond, Eindhoven (NL); VAN DEN BRINK, Johan Samuel, Eindhoven (NL)
(74) Representative: Philips Intellectual Property & Standards

(57) **Abstract**

The present invention relates to a patient engagement tool (PET) for use during magnetic resonance imaging (MRI) procedures. The PET comprises a PET body and at least one MRI accessory integrated within the PET body. This integration allows the PET to serve as both a functional MRI accessory and a tool for engaging patients intuitively during the procedure. By combining these elements, the PET enhances the MRI process by improving patient comfort and compliance while maintaining compatibility with the MRI environment.

## Description

### FIELD OF THE INVENTION

The present invention generally relates to a patient engagement tool (PET) for a patient undergoing a magnetic resonance imaging (MRI) procedure and to a magnetic resonance imaging method.

### BACKGROUND OF THE INVENTION

Diagnostic magnetic resonance imaging procedures involve scanning a patient for, usually, a prolonged period in which the patient is scanned in a tight tunnel-like area. Coil elements are encasing or covering parts of the body and the scan involves loud, unusual and unpredictable noises. The patient must remain still during the examination and, in some cases, needs to hold their breath (e.g. for cardiac scans).

This may cause anxiety and discomfort for patients, which may lead to motion artifacts or incorrectly performed tasks that may prevent a reliable diagnosis or cause rescans. In severe cases anxiety and its effects may be so severe that it leads to aborted scans which also would require rescans and will cause the patient having to go through the stressful procedure again. And also, this is a large burden on patient throughput time and scheduling and on waiting times for other patients.

The traditional approach to reduce anxiety in patients, such as providing training or guidance before and during the scan, using calming techniques, providing an adapted environment, administering sedatives or even using restraints may not be effective or desired for all patients. This is particularly relevant for pediatric patients, claustrophobic patients and, particularly, a growing number of elderly patients with cognitive decline, such as Alzheimer's symptoms or other types of dementia.

Furthermore, pediatric and cognitively impaired patients (elderly or younger) may also not be able to correctly understand or follow instructions, such as remaining still in the same position or follow a breathing guidance instruction for breath holds.

The burden on hospital MRI resources is expected to grow as more MRI procedures are needed due to the emergence of new treatment options at early stages of cognitive decline and the need for continued monitoring during treatment. It is therefore desirable to perform MRI procedures with a higher 'first time right' result.

To enhance the chance of a successful MRI procedure pediatric patients may be trained by using game-like training apps, such as disclosed in US2023368694A1, or with toy-like scan simulators, such as the so-called kitten-scanner, which comprises a miniature mock-up of a diagnostic scanner and patient-simulating dolls and toys equipped with identifiers that are recognized by the miniature scanner, for instance as disclosed in US2009258335A1. These solutions make pediatric patients familiar, in a playful manner, with the equipment and procedure and will reduce anxiety when confronted with the actual imaging device. A number of pediatric patients will still experience higher levels of stress when they are brought into the MRI room and device and/or may not exactly remember their training, particularly if their parents or guides are not able to join them. It would be beneficial to have continued reduced anxiety and improved behavior or compliance just before and during the MRI procedure. And, for patients with cognitive decline, such as Alzheimer's Disease, preprocedural training methods may not be effective as training learnings are not understood or recalled fully or even at all.

As such it would be desirable if patients who are not capable of following or retaining training, guidance or instructions can be engaged intuitively and in real time just before and during the scan procedure to reduce anxiety and/or provide guidance.

### SUMMARY OF THE INVENTION

An embodiment of the claimed invention relates to a patient engagement tool (PET) comprising a PET body and at least one MRI accessory integrated within the PET body. This integrated patient engagement features into an MRI accessory, combining two elements for an MRI procedure on a single patient in a single device, which reduces set-up time for the MRI staff. In the context of the claimed invention an MRI accessory is defined as a device or component that is compatible for use in an MRI environment and is known to be used in the context of an MRI scanner, such as MRI coils, nurse calls, sound devices, cameras, sensors (biometric, location, motion, vibration, etc.), (passive) ultrasound devices, etc.

According to a preferred embodiment of the claimed invention the MRI accessory is configured to enhance patient engagement and/or facilitate the MRI procedure. This improves patient comfort and compliance during MRI procedures, reducing the likelihood of motion artifacts or aborted scans.

According to a preferred embodiment of the claimed invention the MRI accessory includes at least one of a coil element, a patient guidance element (e.g., breathing guidance), a sound device, a nurse call, or a biofeedback sensor. This offers a versatile range of features to address patient anxiety, provide guidance, and monitor physiological parameters, enhancing the overall MRI experience.

According to a preferred embodiment of the claimed invention the MRI coil element is positioned near a surface of the PET body, configured to rest on an anatomical region of the patient during the MRI procedure. This ensures that the PET is on or near the patient to allow for engagement between the PET and the patient. Preferably the PET is positioned on an anatomical region of the patient that is to be imaged. This ensures optimal placement of a coil and/or other components for high-quality imaging while maintaining patient comfort.

According to a preferred embodiment of the claimed invention the MRI coil element can be selected from various types, including receive coils, transmit coils, anatomy-specific coils, and multi-channel coils. This provides flexibility in adapting the PET to different imaging needs and anatomical regions, improving diagnostic accuracy.

According to a preferred embodiment of the claimed invention the PET body is configured to resemble a creature (e.g., an animal, human, or fantasy creature) with an exterior designed to mimic the appearance and tactile feel of the creature. This creates a comforting and familiar interaction for patients, particularly for pediatric and cognitively impaired individuals, reducing anxiety.

According to a preferred embodiment of the claimed invention the PET body includes actuators configured to cause movements, such as chest, head, or limb motions, or vibrations. This engages the patient intuitively through realistic movements, improving compliance and reducing stress.

According to a preferred embodiment of the claimed invention the actuators are configured to simulate a breathing motion of the creature. This enhances the realism of the creature and may encourages the patient to synchronize their breathing with the PET, aiding in procedures that require specific breathing patterns.

According to a preferred embodiment of the claimed invention the actuators are programmed or controlled to simulate a breathing motion that corresponds to a preferred or required breathing pattern of the patient. This enhances procedural accuracy by guiding the patient to maintain the preferred or required breathing pattern for optimal imaging.

According to a preferred embodiment of the claimed invention the actuators include cavities filled with liquid or gas, configured to expand or contract by hydraulic or pneumatic means, or by a solid, such as pellets, balls, that are movable to and from the cavities. This provides a realistic and safe mechanism for simulating breathing or other movements, enhancing patient engagement that is compatible with MRI procedures.

According to a preferred embodiment of the claimed invention the PET body includes a sound device for providing sounds associated with the creature, such as vocalizations, breathing sounds, or calming noises. This reduces patient anxiety through auditory feedback, creating a calming and immersive experience.

The claimed invention further relates to a method for performing an MRI procedure using the PET, including positioning the PET on or near the patient and performing the MRI procedure while the PET remains in position. This integrates the PET seamlessly into the MRI workflow, improving patient comfort and procedural success rates.

According to a preferred embodiment of the claimed invention the method includes determining a preferred or required breathing pattern of the patient and controlling the actuators to simulate a corresponding motion of the PET. This guides the patient to maintain the preferred or required breathing pattern, reducing motion artifacts and improving imaging quality.

According to a preferred embodiment of the claimed invention the method includes providing sounds during or around the MRI procedure that are associated with the PET, such as calming, distracting, or instructional sounds. This increases realism of the PET and enhances the patient's emotional state and compliance through auditory engagement, reducing stress and improving procedural outcomes.

According to a preferred embodiment of the claimed invention the method includes using the nurse call mechanism to communicate with the patient or pause/abort the scan if the patient signals discomfort. This ensures patient safety and comfort by allowing them to signal for assistance during the procedure.

From the above it can be summarized that the PET as claimed and the associated method of using the PET as claimed has several advantages over traditional methods for reducing patient anxiety during MRI procedures, including:
Enhanced Patient Engagement: A design resembling a creature and interactive features of the PET provide a comforting and intuitive experience for the patient.
Improved Compliance: Simulated breathing motions and auditory feedback help patients follow procedural instructions, such as maintaining a specific breathing pattern.
Reduced Anxiety: The PET's calming features, such as tactile interaction, sounds, and movements, reduce anxiety, particularly in pediatric and cognitively impaired patients.
MRI Compatibility: The PET is constructed to be fully compatible with the MRI environment, ensuring safety and functionality during the procedure.
Real-Time Feedback: Biofeedback sensors provide real-time physiological data, enabling the operator to adjust the procedure as needed.
Safety Features: The nurse call mechanism ensures that patients can signal discomfort or request assistance, enhancing safety and comfort.

### BRIEF DESCRIPTION OF THE DRAWINGS

The present invention is illustrated by drawings of which:
Fig. 1 illustrates a schematic representation of the patient engagement tool (PET) according to the present invention.
Fig. 2 depicts the operational setup of the patient engagement tool (PET) during a magnetic resonance imaging (MRI) procedure.
Fig. 3 depicts a flowchart of a method for performing an MRI procedure using the PET.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may not be according to scale.

### DETAILED DESCRIPTION OF THE INVENTION

The present invention relates to a patient engagement tool (PET) designed to enhance patient comfort and engagement during magnetic resonance imaging (MRI) procedures. The PET is particularly beneficial for patients who experience anxiety, discomfort, or difficulty complying with procedural requirements, such as pediatric patients, elderly patients with cognitive decline (e.g., Alzheimer's disease), and claustrophobic individuals. By integrating MRI-compatible components into a robotic device resembling a creature, the PET provides a calming, intuitive, and interactive experience that reduces anxiety, improves compliance, and facilitates successful MRI procedures.

The PET body 1 is designed to resemble a creature, such as an animal, human, or fantasy figure, to create a comforting and familiar interaction for patients. For example, the PET body 1 may take the form of a cat, dog, or baby seal, with an exterior that mimics the appearance and tactile feel of the chosen creature. This exterior may include features such as fur, skin, feathers, or clothing, depending on the creature being represented. The exterior may be removable from the inner part of the PET body 1 for instance to clean the exterior after use, to replace the exterior in case of damage, to customize the PET body 1 to a different creature or for easy access to the parts inside the PET body 1, e.g. for replacement, upgrade or maintenance.

For pediatric patients, the PET body 1 could resemble a playful animal, such as a puppy or fantasy creature, such as a cartoon-like dragon or may resemble a popular cartoon figure, to evoke a sense of fun and reduce anxiety.

For (elderly) patients with cognitive decline, the PET body 1 could take the form of a realistic cat or dog, leveraging the calming effects of pet therapy often used in dementia care, as is for instance known from Petersen et.al. in Journal of Alzheimer's Disease, vol. 55, no. 2, pp. 569-574, 2017. Such known robotic pets are plush toys, such as cats or dogs, configured to act and look realistically by looking and feeling realistic and mimicking pet behavior, such as purring or wagging a tail, amongst others by using robotic actuators and sound effects.

Fantasy creatures, such as unicorns or robots, may also be used to appeal to specific patient preferences or to create a sense of distraction and engagement. By resembling a creature, the PET provides an intuitive and emotionally supportive experience, helping patients feel at ease during the MRI procedure while simultaneously improving compliance and reducing stress.

The PET serves a dual purpose: enhancing patient engagement and facilitating the MRI procedure. These PET includes an MRI accessory, for instance MRI coil elements 11, a nurse call 18 and/or a biofeedback sensor 17, as well as actuators 12, 13 and/or a sound device 16 to make the PET move and provide sound. The PET is positioned on or near the patient 3 during the MRI procedure, engaging the patient 3 while remaining compatible with the MRI environment. To ensure continued proper positioning, the PET can be held in position relative to the patient by straps, clips, Velcro and the like.

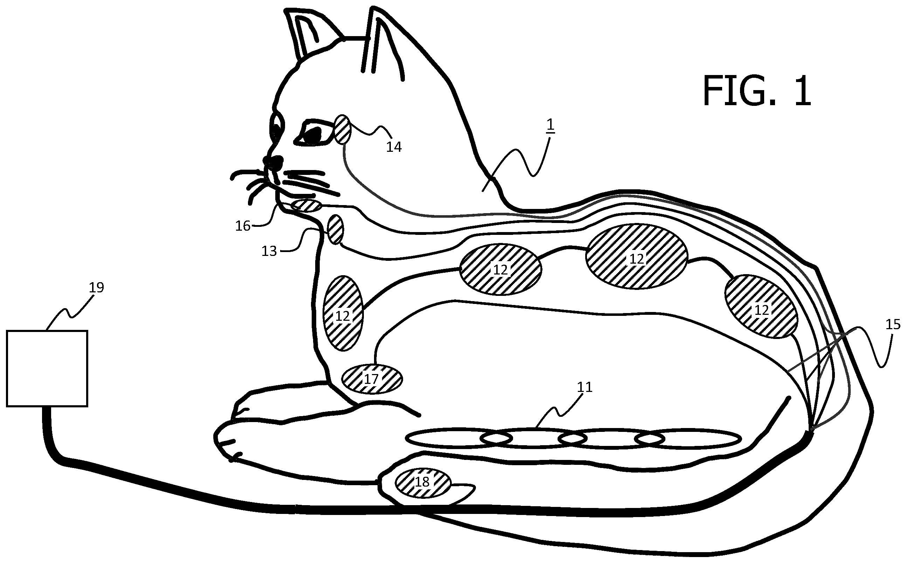

An example of a PET is shown in Fig. 1 in which the PET body 1 is shaped like a cat.

In this example the PET incorporates MRI coil elements 11, such as receive coils, transmit coils, or receive-transmit coils, which are positioned near the surface of the PET body 1. These coils 11 are configured to rest on specific anatomical regions of the patient 3, such as the abdomen, heart, or shoulder, to acquire MRI images. The coil elements 11 may include rigid, flexible, multi-channel, or anatomy-specific designs to optimize imaging performance.

The exemplary PET of Fig. 1 further includes actuators 12, 13 that simulate creature-like movements, such as chest movements to simulate breathing, head or limb motions, and vibrations. One or more body actuators 12 are positioned in a front section, a top section or elsewhere in the PET may simulate movements associated with the PET, including breathing motions. A vibrational actuator 13 may be positioned to generate, for instance, purring sounds or other calming vibrations. Further or more actuators may be implemented or conceived to simulate more precise movements or further movements, such as blinking, tail wagging, limb movements, mouth motions, etc.

The actuators are controlled by a PET control 19 that may be positioned outside or inside the PET body 1. The PET control 19 manages the operation of the integrated components, including the actuators 12, 13,14, sound device 16 and biofeedback sensors 17. Also the nurse call 18 may be connected to the PET control 19. The PET control 19 may be programmed or controlled to actuate individual or combined actuators 12, 13 via actuator control lines 15 and are designed to engage the patient intuitively. For example, the PET can simulate a breathing motion that corresponds to a preferred or required breathing pattern required for the MRI procedure, encouraging the patient to synchronize their breathing with the PET. The PET control 19 may interface with the MRI control system 21 to synchronize the PET's operation with the imaging procedure. The control lines 15 may be led into a guiding sleeve that extends from the PET body 1 to the PET control 19, thereby making a simple, sturdy connection between the PET control 19 and elements within the PET body 1. In an alternative example, some or all the actuators 12, 13 may be controlled wirelessly with MR-compatible wireless control means.

For safety considerations and MRI compatibility the PET may be configured to be controlled and operating in a frequency dependent manner.

A sound device 16, such as an MRI-compatible loudspeaker, may be integrated inside the PET body 1. The sound device 16 may be used to provide auditory feedback, such as calming sounds (e.g., purring, breathing, or vocalizations) or instructional sounds. These sounds reduce patient anxiety and improve compliance with procedural instructions. It may also be used to provide instructions or communicate with the patient 3. To avoid unnecessary patient anxiety, this should preferably be done in a manner that makes sense with respect to the creature mimicked by the PET.

The sound device 16 may provide sounds that intuitively guide the patient, for instance to a desired breathing pattern or heart rate pattern. For instance the sound device 16 may provide breathing sounds in a desired or preferred breathing pattern for the procedure that the patient will intuitively synchronize too, especially if combined with breathing motions of the PET body 1 caused by any of the actuators 12, 13. Alternatively or additionally, if here is a desired heart rate for a patient, for instance because of requirements of the exam. For instance, a higher or lower heart rate may be desired to improve a cardiac exam. If the heart rate is too high or low a cardiac sequence may take a long time, or a patient may need to have a scan in stress and rest conditions and the heart rate may need to be increased or decreased. Stress may also increase the heart rate to undesired levels and providing regular and/or lowered cardiac sounds from the PET may have a calming, and heart rate reducing, effect on the patient. To influence the patient's heart rate, the sound device 16, potentially in combination with or alternative to simulated cardiac motion provided by actuators 12, 13 in the PET body 1, may synchronize the patient's heartbeat, at least to some extent, by slowing down or speeding up the simulated heartrate from the PET. This may also be executed in combination with breathing synchronization.

The PET body 1 may include one or more sensors 17 for sensing data from the patient. For instance, the PET body 1 may include a biofeedback sensor to measure physiological parameters of the patient, such as skin conductivity, respiratory activity or heart rate. These sensors provide real-time feedback to the MRI operator, enabling adjustments to the procedure or the PET's operation based on the patient's condition. Other exemplary sensors may be motion sensors, such as accelerometers, for detecting motion of the patient or temperature sensors to detect the temperature of the patient, particularly if a patient would experience overheating.

In a further example a nurse call mechanism 18 is integrated into an accessible part of the PET body, such as a tail, limbs or ears of the creature. This mechanism 18 allows the patient to signal discomfort or request assistance during the MRI procedure, ensuring safety and comfort. Common MRI-compatible nurse call mechanisms may be used for this purpose.

The PET may include an MRI-compatible camera 14 that is directed towards the patient 3 when the PET is placed in the correct position, e.g. as a creature facing the patient 3. The camera 14 may for instance be positioned in or behind the (transparent) eyes of the creature shaped PET body 1, as is shown in Fig. 1. The camera 14 may be used to monitor the patient 3 while in the bore of an imaging device 2 during a procedure, particularly if a view of any present out-of-bore camera is obstructed by the imaging device, coils or the PET itself. As such an emotional state or undesired motion may be detected and cause staff or the PET to provide instructions, calming or encouraging to the patient 3. If two cameras 14 are installed, for instance one in or behind each eye, a 3D depth image may be obtained of the patient 3 that allows for even more precise position or motion determination.

The PET body 1, including all internal elements, such as the actuators 12, 13 are fully constructed of MRI-compatible materials. As ferromagnetic metal elements are not suitable for use in an MRI environment, such materials, for instance regular electronic control lines for the actuators 12, 13 is not recommended. To be able to control the actuators 12, 13 MRI-compatible solutions are preferably implemented. An exemplary solution is that the actuators 12, 13 include cavities that are filled with a liquid or gas. These may expand or contract by hydraulic or pneumatic means. The control lines 15 are then hydraulic or pneumatic control lines controlled by the PET control 19. Alternatively, the control lines comprises a solid material, such as pellets or balls of MRI-compatible material, that are movable to and from the cavities and thereby creating a motion of the actuator. The liquid, gaseous or solid material used to activate the actuators should be chosen such that it does not interfere with the MRI imaging.

The PET control 109 may include logic to provide interactive feedback to actions by the patient 3. For instance, when the PET is stroked, the PET control 19 may activate actuators or a sound device 16 to provide an appropriate or desired response. Such responses may be programmed based on known suitable responses to patient actions or may be generated through artificial intelligence trained on such responses. This may be a generative artificial intelligence that is particularly adaptable in providing the most appropriate response.

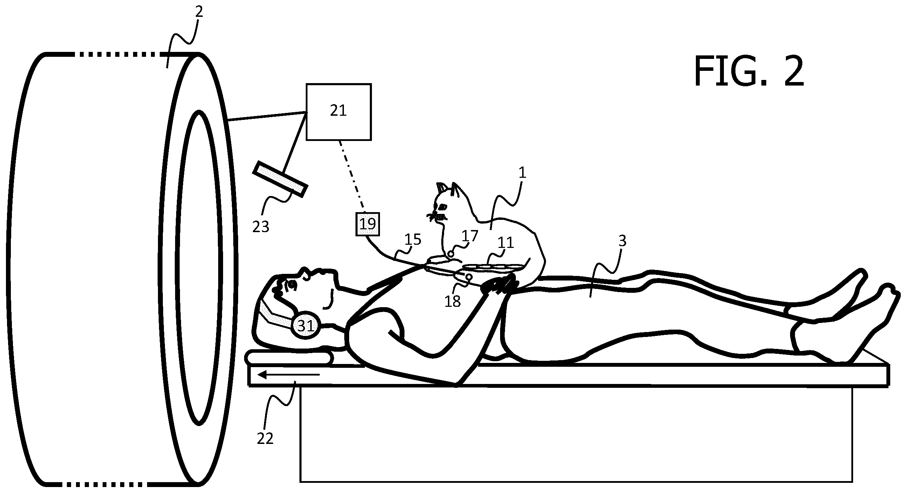

Fig. 2 shows an illustration of the PET in use with a patient 3 about to undergo a diagnostic scan in a magnetic resonance imaging device 2.

The patient lies on a patient support 22 that will move into the bore of the MRI scanner 2 for the imaging procedure. The PET is positioned on the belly of the patient 3, in this case on the belly. In this position the MRI coil elements 11 near the lower surface inside the PET body 1, are positioned optimally for an abdominal scan. Alternate positions of the PET body 1 on the are also conceivable for other anatomical regions of interest, e.g. a position on the chest, which allows for cardiac or lung imaging or on the arm for an elbow or hand scan.

In this example a nurse call button 18 is integrated in the tail section of the PET body 1 that is easily accessible to the patient 3. The patient wears ear protection 31 in the form of headphones that protect the patient from loud scan noises, but also allow for providing calming or instructional sounds to the patient or by providing appropriate or functional sounds from the PET to the patient 3. This may be used as an alternative or in addition to a sound device 16 in the PET body 1, as this may be of no use in the very loud environment of an MRI in operation.

The PET is connected to the PET control 19 through control lines 15 (in this example guided through a guide sleeve). The PET control may also be connected to the MRI control 21 to allow for interaction between the PET and the MRI 2, for instance in adapting image acquisition (e.g. gated acquisition) to a breathing pattern of the patient induced by or sensed (for instance with a sensor 17 inside the PET body) by the PET.

A display 23 may provide visual feedback, such as a graphical representation of a preferred or required breathing pattern, to guide the patient during the procedure. The display may be connected to the MRI control 21 to allow for gated MRI acquisition based on the provided breathing instructions.

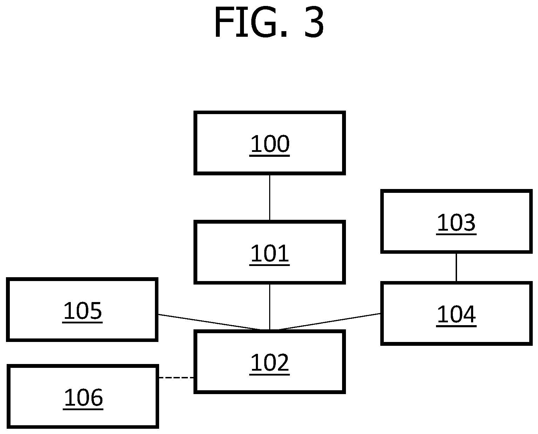

Fig. 3 shows a schematic flowchart of an exemplary method to perform an MRI scan using the PET.

First, the patient 3 is prepared and positioned on a patient support 22 and provided 100 with the PET, either at this stage or earlier, which is then positioned 101 on or near the patient 3, ensuring proper alignment of the MRI coil elements 11 to the anatomical region of interest to be scanned. Before and during the scan the PET engages the patient 3 through simulated movements, sounds, and tactile interaction, reducing anxiety and encouraging compliance. The patient 3 is moved into the MRI device 2 and the MRI procedure is conducted 102 while the PET remains in position (optionally fixed by straps or pins or other fixtures) , with its integrated components facilitating imaging and patient engagement.

In a further example a preferred or required breathing pattern of the patient is determined 103. This may be done by measuring based on a patient's breathing cycle (e.g. by using a sensor 17 embedded in the PET body, training the patient towards a specific breathing cycle or a breathing pattern determined to be suitable or required for the MRI procedure. Next the PET control 19 controls 104 the appropriate actuators 12, 13 and/or sound device 16 and/or ear protection 31 to cause a motion in the PET and/or generate sounds to guide the patient 3 towards the preferred or required breathing pattern.

In a further example the PET provides 105 sounds to the patient 3, using the sound device 16 and/or ear protection 31, during or around the MRI procedure that are sounds associated with the PET, for instance calming sounds, distracting sounds, and/or instructional sounds.

In a further example, the patent may communicate 106 with radiology staff through the ear protection 31 and /or using a nurse call 18 embedded in the PET body 1. The nurse call 18 may be configured to automatically abort or pause the scan when the patient 3 uses it, particularly if the patient would use it in a specific manner, e.g. by using it repetitively within a very short time.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A patient engagement tool (PET) for a patient (3) undergoing a magnetic resonance imaging (MRI) procedure, comprising:
- a PET body (1); and
- at least one MRI accessory (12, 13, 14, 16, 17) integrated within the PET body.

2. The PET according to claim 1, wherein the MRI accessory is configured to provide patient engagement and/or facilitate the MRI procedure.

3. The PET according to claim 1 or 2, wherein the MRI accessory includes at least one of a coil element (11), a patient guidance element, for instance a breathing guidance element, a nurse call (18), a camera (14), or a biofeedback sensor (17) configured to measure patient physiological parameters, such as respiratory activity or heart rate.

4. The PET according to any of claims 1 to 3, wherein the MRI accessory includes an MRI coil element that is located near a surface of the PET body, wherein the surface is configured to rest on an anatomical region of the patient during the MRI procedure, preferably an anatomical region of the patient that is to be imaged.

5. The PET according to any of claims 1 to 4, wherein the MRI accessory includes an MRI coil element selected from one or more of a receive coil element, a transmit coil element, a receive-transmit coil element, an anatomy-specific coil element, a rigid coil element, a flexible coil element, a multi-channel coil element, a parallel imaging coil element, a segmented coil element, and/or any other MRI coil element common in the field of MRI and combinations thereof.

6. The PET according to any of claims 1 to 5, wherein the PET body is configured to resemble a creature, including an animal, human, or fantasy creature, and comprises a PET body exterior designed to mimic the appearance and tactile feel of the creature, including features such as fur, skin, hair, feathers, or clothing, preferably a PET body exterior that is removable.

7. The PET according to claim 6, wherein the PET body includes actuators (12, 13) configured to cause at least one movement of the PET body or parts thereof, particularly wherein the at least one movement is a movement associated with the creature, such as chest, head, and/or limb movements or vibrational movements.

8. The PET according to claim 7, wherein the actuators are arranged and configured to cause at least one movement to simulate a breathing motion of the creature.

9. The PET according to claim 8, wherein the actuators are programmed or controlled to cause a breathing motion of the creature that corresponds to a breathing pattern of the patient that is preferred or required for the MRI procedure.

10. The PET according to any of claims 7 to 9, wherein the actuators include cavities filled with a liquid or gas that are configured to expand or contract by hydraulic or pneumatic means, or by a solid, such as pellets, balls, that are movable to and from the cavities.

11. The PET according to any of claims 6 to 10, wherein the PET body includes a sound device (16) for providing sounds associated with the creature, such as, for instance, vocal sounds, breathing sounds, cardiac sounds, or sounds associated with movements and/or vibrations, and/or for providing communication of staff to the patient.

12. A magnetic resonance imaging (MRI) method, including the steps of:
- providing (100) a patient (3) undergoing a magnetic resonance imaging (MRI) procedure with a patient engagement tool (PET) according to any of claims 1 to 11;
- positioning (101) the PET on or near the patient (3) such that the PET engages with the patient and facilitates the MRI procedure; and
- performing (102) the MRI procedure while the PET remains in position.

13. The MRI method according to claim 12, wherein the PET comprises actuators (12, 13) as claimed in any of claims 7 to 10, comprising the further steps of:
- determining (103) a preferred or required breathing pattern of the patient for the MRI procedure; and
- manually or automatically controlling (104) the actuators to cause a motion of the PET body (1) that corresponds to the preferred or required breathing pattern of the patient.

14. The MRI method according to any of claims 12 to 14, further comprising the step of
- providing sounds (105) during or around the MRI procedure that are sounds associated with the PET or communication from staff, for instance calming sounds, distracting sounds, and/or instructional sounds.

15. The MRI method according to any of claims 12 to 15, wherein the PET comprises a nurse call (18) as claimed in claim 3, further comprising the step of:
- communicating (106) with the patient or aborting or pausing the scan when the patient uses the nurse call.
